# EUROPEAN PATENT APPLICATION

(11) **EP 1 350 509 A1**
(43) Date of publication of application: **08.10.2003**
(21) Application number: 01982734.4
(22) Date of filing: 02.11.2001
(51) Int. Cl.: A61K 31/16, A61K 39/39, A61P 37/02

(54) **MUCOSAL IMMUNOMODULATOR AND USE THEREOF**

(30) Priority: 07.11.2000 JP 2000339753; 18.07.2001 JP 2001217899
(71) Applicant: KABUSHIKI KAISHA HAYASHIBARA SEIBUTSU KAGAKU KENKYUJO, Okayama-shi Okayama 700-0907 (JP)
(72) Inventor: ARAI, Norie, K.K.Hayashib.Seibutsu K.Kenky., Okayama-shi, Okayama 700-0907 (JP); HANAYA, Toshiharu, K.K.Hayashib.Seibutsu K.Kenky., Okayama-shi, Okayama 700-0907 (JP); ARAI, Shigeyuki, K.K.Hayashib.Seibutsu K.Kenky., Okayama-shi, Okayama 700-0907 (JP); KURIMOTO, Masashi, K.K.Hayashi.Seibutsu K.Kenky., Okayama-shi, Okayama 700-0907 (JP)
(74) Representative: Daniels, Jeffrey Nicholas
(86) International application number: JP0109646
(87) International publication number: WO02038146

(57) **Abstract**

The object of the present invention is to provide a mucosal immunoregulatory agent which has neither excessive stress nor care for side effects even when administrated repeatedly, and solves the objected by providing the mucosal immunoregulatory agent comprising trehalose.

## Description

### Technical Field

The present invention relates to a mucosal immunoregulatory agent using trehalose as an effective ingredient, which is prepared into foods, beverages, pharmaceuticals, toiletries and feeds including pet foods.

### Background Art

Higher animals including humans commonly have immune functions which are to protect them from the infection by pathogenic organisms such as bacteria, viruses and parasites. In the second half of the 20th century, studies on humoral immunity, where antibodies and cytokines are involved in as major factors, have been rapidly progressed, resulting in the development of various medicines.

Mucosal immunity is a system which is to prevent the infection by pathogenic bacteria or the like. Several types of immunocompetent cells which are present in mucosae, maintain the function of such system. The immunocompetent cells produce immunoglobulin A (IgA). Since IgA shares 60% or more of the total amount of globulins, the regulation of its production would be important to maintain healthy conditions.

However, a remarkable increment in incidence of food poisoning, food allergy and polinosis may suggest that the mucosal immunity of persons in modernized society may be easily damaged. Factors as listed for such a phenomenon may be stresses, irregular daily life, inappropriate nutrition, abuse of medicines, and excessive sanitation. Thus, effective means for regulating mucosal immunity have been in great expectation.

### Disclosure

The present invention is to provide an agent which would regulate mucosal immune function without causing have neither excessive stress nor care for side effects during repeating administration when administrated through oral route.

To attain this objective, the present inventors screened a variety of substances, resulting in a novel finding that trehalose, a non-reducing disaccharide, dose remarkably regulate the production of IgA and cytokines, such as IFN-γ, by immunocompetent cells which are present in mucosae: In particular, trehalose elevates the production of IgA and IFN-γ in unhealthy persons, while in healthy persons, trehalose elevates the production of IgA but reduces IFN-γproduction. Thus, the mucosal immunoregulatory agent according to this invention moderates and maintains the mucosal immunity inherent to humans and animals when taken through oral route. Such an effect is remarkably seen in cells which are present in intestinal mucosa, particularly, those present in the Peyer's patches.

Trehalose, which is used as the effective ingredient in this invention, is a disaccharide where two molecules of glucose are bound each other at their reducing ends. There are three types of isomers with different binding fashions; α,α-trehalose (isomer of this type may be called shortly "trehalose".), α,β-trehalose (or neotrehalose) and β,β-trehalose (or isotrehalose). Trehalose used in this invention is one or more types of these isomers. Particularly, α,α-trehalose can be most favorably used in this invention because it is the most widely distributed in the nature such as bacteria, fungi, algae, insects, crustaceans, in the nature, and also because one can get a desired amount of α,α-trehalose which commercialized with an established technology for mass production. In this invention, trehalose preparations are limited to neither those which have a particular structure or purity nor those which are prepared with a particular method, as long as they exhibit the prescribed effect in the mucosal immunoregulatory agent according to this invention. Trehalose has been incorporated or prepared into various foods, the fact that trehalose would confirm that the agent of this invention would be usable in various forms such as foods, beverages, pharmaceuticals or toiletries with no care for side effects . Since low purity trehalose preparations are lower in cost than high purity trehalose preparations. The former preparations can be advantageously used in feeds and pet foods for animals including domestic animals such as cows, horses and sheep, fowls such as chickens and gooses, and pets such as dogs and cats in addition to use in humans.

Thus, the present invention provides a mucosal immunoregulatory agent which is incorporated or prepared with trehalose as an effective ingredient, to attain the above-mentioned objective.

### Brief Description of Drawing

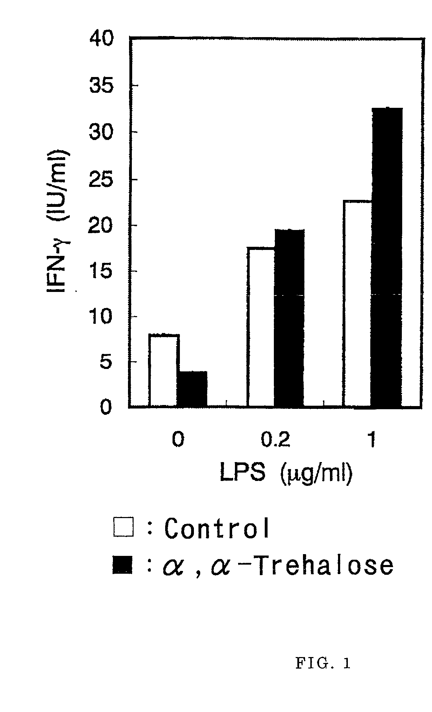

FIG. 1 shows IFN-γ production by immunocompetent cells from the Peyer's patches which has been stimulated with various concentrations of LPS.

### Best Mode for carrying out the Invention

The wording "mucosal immunoregulatory agent" as referred to in this invention means those which contain trehalose as an effective ingredient; such agent exhibits prescribed effects of modulates mucosal immune function in mucosae of digestive organs such as oral-, stomachic-and intestinal-mucosae, particularly, in intestinal mucosae and the Peyer's patches, when administrated through oral pathway.

Trehalose, the effective ingredient in the mucosal regulatory agent according to this invention, may contain additional components inherent to its preparation method, as long as such components do not affect the objective of this invention. An α,α-trehalose product commercialized by Hayashibara Shoji Co. Ltd., Okayama, Japan under the registered trademark of "TREHA" can be advantageously use in the mucosal immunoregulatory agent of this invention.

The structure and function of the Peyer's patches have been clarified in recent years. As reviewed by Hashiguchi et al., "Clinical immunology (Rinsho-Meneki)", Vol. 30, No. 11, pp. 1524-1531 (1998) and Takahashi et al., "Medical Immunology", Vol. 27, No. 5-6, pp. 431-437 (1994). The Peyer's patches, a type of immune tissue, are present on the intestinal periphery and aggregates of lymph modules which have immunocompetent cells such as M cells, B cells, T cells and macrophages. The main function of the Peyer's patches is to produce IgA and cytokines, for example, IFN-γ, interleukin 2 (IL-2) and interleukin 5 (IL-5). Furthermore, as reviewed by Kiyono et al., "Clinical Immunology (Rinsho-Meneki)", Vol. 30, No. 1, pp. 14-19 (1998), it has been clarified that IFN-γ has a regulatory action on IgA production.

The action mechanism of the mucosal immunoregulatory agent of this invention may be explained as follows; when administrated to humans or animals such as domestic animals, fowls and pets through oral pathway, the trehalose in the agent may bind with mucosae of digestive organs such as oral cavity, gullet, stomach, duodenum and small intestine, then, trehalose is hydrolyzed into two molecules of glucose in small intestine.

The mucosal immunoregulatory agent of this invention, which contains trehalose as an effective ingredient, can be used in various forms such as foods, beverages, pharmaceuticals, toiletries, feeds and pet foods, as long as they contain trehalose as an effective ingredient. Above mentioned effect of the mucosal immunoregulatory agent of this invention can be confirmed by the test described below where cytokines and/or antibodies are produced by immunocompetent cells from intestinal mucosa or the Peyer's patches, preferably, those from the Peyer's patches.

The mucosal immunoregulatory agent of this invention can be prepared into a desired form where trehalose, an effective ingredient, is used alone or in combination with one or more ingredients which facilitate the administration of trehalose. The wording "administration through oral pathway" as referred to in this invention means that oral intake can reach to mucosae of digestive organ, those which, therefore, for example, it involved intubation methods which used a stomach tube. The composition, which facilitates oral administration of trehalose, can be provided as foods, beverages, pharmaceuticals, toiletries, feeds or pet foods in solution, suspension, emulsion, cream, paste, powder, or other desired form which are for usual oral intake in addition to intubation feeding. Thus, in case of foods and beverages to facilitate of trehalose, the agent is prepared into compositions along with additional materials and/or ingredients which are used in usual foods and beverages such as water, alcohols, starches, proteins, fibers, saccharides, lipids, vitamins, minerals, flavors, colorants, sweeteners, seasonings, spices, stabilizers, antioxidants and preservatives. Furthermore, such compositions can be arbitrarily used in combination with one or more health food materials such as sugars for Bifidus factor, powder milk, resolved products of milk protein (casein calcium peptide, casein phosphor-peptide), lactoferin, soybean isoflavon, blood powder, bone powder, shell powder and coral powder. The foods can be a form like a liquid diet for intubation. Furthermore, when the compositions involved in pharmaceuticals or toiletries, for example, one or one more of carriers, excipients, dilution agents and stabilizers as ingredients to ease oral administration. If necessary, the composition may be mixed with one or one more calcium agents such as calcium lactate, calcium glycerophosphate, calcium hydrophosphate and calcium L-asparaginate, and others such as sedative drugs, antiphlogistic drugs, active type vitamin D drugs, vitamin K drugs, calcitonin drugs, estrogen drugs and protein anabolism hormone drugs, that are used to treat for various diseases. Furthermore, when the composition is incorporated in feeds and pet foods, it can be produce to added and mix a proper quantity of trehalose to normally used feed and pet foods. Furthermore, specially, when the concentration of the mucosal immunoregulatory agent of this invention in order to raise its concentration in intestine, or when some ingredients which would be susceptible to gastric juice are suitably used, it can be used in the form of a tablet or granule coated by enteric resolvable materials. The mucosal immunoregulatory agents of this invention usually comprises 0.1%(w/w) or more of trehalose, desirably, 1%(w/w) or more of trehalose, depending on final use.

A preferred example and dosage of the mucosal immunoregulatory agent of this invention for human use are described below. The agent can be used with the purpose of preventing diseases and improving therapeutic effects. In particular, the agent can exhibit effective action against the diseases due to viruses such as hepatitis A virus, poliovirus and rotavirus, bacteria such as cholera, dysentery, typhoid, salmonella, campylobacter, *Pseudomonas pseudomallei. Vibrio parahaemolytious* and *Brucella,* parasites such as broad tapeworm, Yokokawa fluke, oriental liver fluke, echinostoma, lung fluke, anisakis, gnathostoma, *Anglostrongylus cantonensis, Entamoeba histolytica, Cryptosporidium,* malaria and microfilaria, or an allergen, which inhabit in foods. In order to prevent such diseases , the mucosal immunoregulatory agent of this invention is usually prepared into a food or beverage which facilitates intake of the agent through oral pathway. The agent can be formed pharmaceutical such as a syrup, powder, granule, tablet or capsule in order to treat diseases or improve the symptom of diseases. The mucosal immunoregulatory agent of the present invention can be administrated with 0.5 to 100g per adult a day of trehalose by daily or one to five times per week.

While, in the case of administering to animals, the dosage can be decided in view of the body weight of the animals similarly as in humans as described above. That is, the mucosal immunoregulatory agent can be administrated with 0.01 to 2g per kg body weight per day, desirably, 0.02 to 1g per kg body weight per day.

Furthermore, trehalose is used with ease because it has been used in the field of foods and has been confirmed safe when administered orally.

The present invention will be explained in detail with reference to the following experiments.

### Experiment 1

Effect on response of immunocompetent cells from the Peyer's patches (1)

The effect on response of immunocompetent cells with trehalose administration was evaluated based on the production level of IFN-y as a merkmal when the immunocompetent cells from the Peyer's patches were stimulated with lipopolysaccharide (LPS). IFN-γ is a cytokine which also improves the production of IgA as described above. α,α-Trehalose crystalline powder (registered trademark of "TREHA", commercialized by Hayashibara Shoji Co. Ltd., Okayama, Japan) was dissolved in distilled water and sterilized, and then administrated into male C3H/HeN, five weeks aged mice (ten mice in each group) through oral pathway for five days by one time per day with a dosage of 1g/kg mouse body weight of α,α-trehalose weight. Non-α,α-trehalose administrated mice as a control were administrated with an equal volume of distilled water. All the mice were bred under a clean environment, and freely fed with a sterilized solid fodder and water. On the day after the last administration day, the mice were anatomized and picked up all their Peyer's patches which were then cut into pieces and treated with 0.2%(w/v) of a collagenase solution at 37°C for 30 minutes, passed through a cell strainer to remove non-digestive materials, and centrifuged to obtain a pellet. The pellet was suspended in a 45%(v/v) percol solution, and the suspension was put on a 75%(v/v) percol solution, and centrifuged. Then, immunocompetent cells in an inter layer of percol were recovered and suspended in RPMI1640 medium with 10% of fetal calf serum and 5X10⁻⁵ mol/1 of 2-mercaptetanol, and then mixed with 0, 0.2 or 1µg/ml of LPS and adjusted to give a final concentration of 2X10⁶ cells/ml. The cells were incubated for two days in an incubator maintained at 37°C and 5% CO₂, after that, the IFN-γ concentration in each culture was measured by a conventional enzyme immunoassay. In addition, in this experiment, using a standard mouse IFN-γ (GgO2-901-533) obtained from the National Institutes of Health, the IFN-γ activity was expressed in terms of the international standard unit (IU). The results are in FIG. 1.

As obviously from the result in FIG. 1, the competent cells from the Peyer's patches from α,α-trehalose treated mice had a stronger tendency of increasing production of IFN-γ under the LPS stimulation than those of the non-α,α-trehalose treated mice. This result would suggest that the oral administration of α,α-trehalose effectively prevents and treats diseases caused by the infection of bacteria because it may increase immune response of the immunocompetent cells against bacterial stimulation. While, the oral administration of α,α-trehalose would be expected to excessively increase IFN-γ production under the normal condition because there is tendency to decrease IFN-γ production with no LPS stimulation.

### Experiment 2

Effect on response of immunocompetent cells from the Peyer's patches (2)

The effect on the response of immunocompetent cells with trehalose administration was evaluated by the production level of IL-2, IFN-γ, and IgA as a merkmal when the immunocompetent cells from the Peyer's patches stimulated with lipopolysaccharide (LPS) or Concanavalin A (Con A). Specifically, 0.1g/ mouse body weight of α,α-trehalose was administrated into female ddY mice through oral administration for four weeks with five times per a week, and non- α,α -trehalose administrated mice were used as a control. All the mice were anatomized and picked up all the Peyer's patches, which were then cut into pieces and treated with 0.2%(w/v) of collagenase solution at 37°C for 30 minutes, passed through a cell strainer to remove non-digestive materials, and centrifuged to obtain a pellet. The pellet was suspended with a 45%(v/v) percol solution, and the suspension was put on a 75%(v/v) percol solution and centrifuged. Then, immunocompetent cells in an inter layer of percol were recovered and suspended in RPMI1640 medium with 10% of fetal calf serum and 5X10⁻⁵ mol/l of 2-mercaptethanol. The resulting suspension was divided into three groups, and the two groups of which were mixed with 2µg/ml of LPS or 5µg/ml of Con A and adjusted a final concentration to 1X10⁶ cells/ml. The resulting group as a control was not received with the above stimulation. They were incubated for three days in an incubator maintained at 37°C and 5% CO₂. Thereafter, the concentrations of the IL-2, IFN-γ and IgA concentration in the resulting cultures were measured by a conventional immunoassay method in Experiment 1. The results are in Table 1.

**Table 1**

| Samples | Stimulation | lL-2(pg) | IFN-γ (IU/ml) | lgA(ng/ml) |
|---|---|---|---|---|
| α, α- Trehalose | LPS | 115 | 4.5 | 2305 |
| Control | LPS | 56 | 0.6 | 721 |
| α, α - Trehalose | ConA | Under 50 | 6.4 | 1671 |
| Control | ConA | 53 | 3.6 | 970 |
| α, α - Trehalose | Non | Under 50 | Under 0.4 | 719 |
| Control | Non | Under 50 | Under 0.4 | 409 |

As evident from the resulting Table 1, the immunocompetent cells from the Peyer's patches of α,α-trehalose treated mice had a stronger tendency of increasing production of IFN-γ under LPS or Con A stimulation than that of IFN-γ under non-LPS or non-Con A stimulation. The production of IL-2 under LPS stimulation was the same result of IFN-γ. While, production of IgA under the non-stimulation by the Peyer's patches of α,α-trehalose treated mice was kept in high level, and the production was increased under the stimulation. This result would suggest that oral administration of α,α - trehalose is effective in preventing and treating diseases caused from the oral infectious disease because it may be increased the production of cytokines and IgA antibody.

The embodiments according to the mucosal immunoregulatory agent of the present invention are explained in detail.

### Example 1

### Health food

Two parts by weight of a gum base were heated and dissolved until softened, and then mixed with two parts by weight of a maltose powder, four parts by weight of sucrose powder and one part by weight of a crystalline α,α-trehalose powder (registered trademark of "TREHA" commercialized by Hayashibara Shoji Co. Ltd., Okayama, Japan) , and then mixed with small amounts of a mint flavor and a color, and in a conventional manner kneaded and shaped into a gum.

This product is tasty and flavorful and useful as a health food to maintain and improve health because it can regulate the mucosal immune function when eaten.

### Example 2

### Fluid diet

An oral fluid diet was prepared in a usual manner by mixing the following ingredients:

| | |
|---|---|
| Crystalline α,α-trehalose | 1 Part by weight |
| Skim milk | 43 Parts by weight |
| Complete powder milk | 12 Parts by weight |
| Starch syrup | 42 Parts by weight |
| Glucose | 3 Parts by weight |
| Vitamin A | Desired amount |
| Vitamin D | Desired amount |
| Thiamin hydrochloride | Desired amount |
| Riboflavin | Desired amount |
| Pyridoxine hydrochloride | Desired amount |
| Cyanocobalamin | Desired amount |
| Coline hydrogen tartaride | Desired amount |
| Nicotinic acid amide | Desired amount |
| Calcium pantothenate | Desired amount |
| L-Ascorbic acid | Desired amount |
| Tocopherol acetate | Desired amount |
| Ferric sulfate | Desired amount |
| Calcium hydrogen phosphate | Desired amount |
| Gum arabic | Desired amount |

When this product is dissolved in the desired amount of water and administrated though oral to a patient who must not be administered with normal food, this product improves patient's condition because it regulates mucosal immune system. Furthermore, the product can be administered by intubation.

### Example 3

### Health supplement

Forty parts by weight of crystalline α,α-trehalose, 20 parts by weight of a natural coral powder, ten parts by weight of a cow bone powder, ten parts by weight of a yoghurt powder, 12 parts by weight of guar gum, and three parts by weight of vitamin C, and 0.5 part by weight of glycosyl vitamin P were mixed, and granulates by fluidized bed type of granulator as spraying and dropping desired amount of water, according to conventional manner, and then, shattered and made grains, then, powder for making tablets was obtained. To the powder was added three parts by weight of sucrose fatty acid ester as a gross-imparting agent, and then uniformly mixed and tableted by a tableting machine which had mallets of six mm in diameter to obtain tablets comprising trehalose (about 200mg/tablet) were obtained.

This product is tasty and flavorful and useful as a health food to maintain and improve health because it can regulate the mucosal immune function when eaten.

### Example 4

### Enteric-coated tablet

Tablet type of mucosal immunoregulatory agent of this invention, obtained by the method in Example 3, was coated with cellulose acetate to obtain an enteric-coated tablet.

This product, which is not dissolved until reaching the intestinal canal can transfer the effective ingredient to the intestinal canal and exhibit the effect of regulating immune function by intestinal immune system with smaller dosage.

### Example 5

### Health food

A health food as a cheese cracker was prepared by mixing the following ingredients:

| | |
|---|---|
| Flour | 100 Parts by weight |
| Fat | 9 Parts by weight |
| Malt extract | 1.3 Parts by weight |
| Sodium bicarbonate | 0.6 Part by weight |
| Cheese powder | 13 Parts by weight |
| α,α - Trehalose | 2 Parts by weight |
| Sugar | 2 Parts by weight |
| Salt | 1 Part by weight |
| Ammonium carbonate | 0.6 Part by weight |
| Spice | Desired amount |
| Water | 33 Parts by weight |

This product is tasty and flavorful and useful as a health food to maintain and improve health because it can regulate the mucosal immune function when eaten.

### Example 6

### Health food

A green tea extract was obtained by extracting one part by weight of a blended green tea for liquid beverage with 30 parts by weight of 65 °C ion-exchanged water for five minutes. This extract was diluted with ion-exchanged water to give a drinkable concentration, and then mixed with 1%(w/v) of α,α-trehalose and 0.03%(w/v) of L-ascorbic acid to obtain a diluted extract. According to a conventional manner, the extract was filled into an acceptable bottle and sealed off, and sterilized, to obtain a green tea beverage type of health food.

This product is tasty and flavorful and useful as a health food to maintain and improve health because it can regulate the mucosal immune function when eaten.

### Industrial Applicability

The present invention was made based on the finding that orally administered trehalose mediates the mucosal immune system via the action of immunocompetent cells in mucosae, particularly the Peyer's patches. Because of this action, the mucosal immunoregulatory agent of the present invention would demonstrate effects to treat, prevent and alleviate oral epidemic, food allergy or the like which are easily induced by disorder of immune function, and can be usually used with no care because it is substantially free from side effect, normally controls the production of cytokines, and then trehalose as efficient ingredient is very cheep. The present invention with such remarkable effects is an outstandingly significant invention that greatly contributes to this art.

## Claims

1. A mucosal immunoregulatory agent **characterized in that** it comprises trehalose as an effective ingredient.

2. The mucosal immunoregulatory agent as described in claim 1, **characterized in that** said trehalose is α,α-trehalose.

3. The mucosal immunoregulatory agent as described in claim 1 or 2, wherein said agent is a gastrointestinal mucosa.

4. The mucosal immunoregulatory agent as described in claim 3, wherein said gastrointestinal mucosa is an intestinal mucosa.

5. The mucosal immunoregulatory agent as described in any one of claims 1 to 4, which is in the form of a food, beverage or feed.
